Europäisches Patentamt

European Patent Office

Office européen des brevets

(19)

(11) Veröffentlichungsnummer: **0 250 993 B1**

(12) # EUROPÄISCHE PATENTSCHRIFT

(45) Veröffentlichungstag der Patentschrift :
**13.11.91 Patentblatt 91/46**

(51) Int. Cl.$^5$ : **A61C 9/00**

(21) Anmeldenummer : **87108531.2**

(22) Anmeldetag : **12.06.87**

(54) **Verfahren und Vorrichtung zur Bestimmung und Darstellung der dreidimensionalen Gestalt von präparierten Zähnen.**

(30) Priorität : **24.06.86 CH 2512/86**

(43) Veröffentlichungstag der Anmeldung :
**07.01.88 Patentblatt 88/01**

(45) Bekanntmachung des Hinweises auf die
Patenterteilung :
**13.11.91 Patentblatt 91/46**

(84) Benannte Vertragsstaaten :
**AT BE DE FR GB IT NL SE**

(56) Entgegenhaltungen :
**EP-A- 0 040 165
EP-A- 0 054 785
DE-A- 2 936 847
US-A- 3 861 044
US-A- 4 575 805
HOLODONTOGRAPHY, AN INTRODUCTION
TO DENTAL LASER HOLOGRAPHY, USAF, report SAM-TR 74-4, AD-785 191, März 1973 Seite
10**

(73) Patentinhaber : **Brandestini, Marco,
Dr.sc.techn.
Gartenstrasse 10
CH-8702 Zollikon (CH)**
Patentinhaber : **Mörmann, Werner H.,
Dr.med.dent.
Zweiackerstrasse 57
CH-8053 Zürich (CH)**

(72) Erfinder : **Brandestini, Marco, Dr.sc.techn.
Gartenstrasse 10
CH-8702 Zollikon (CH)**
Erfinder : **Mörmann, Werner H., Dr.med.dent.
Zweiackerstrasse 57
CH-8053 Zürich (CH)**

(74) Vertreter : **Blum, Rudolf Emil Ernst et al
c/o E. Blum & Co Patentanwälte Vorderberg 11
CH-8044 Zürich (CH)**

EP 0 250 993 B1

## Beschreibung

Die Erfindung betrifft ein Verfahren zur räumlichen Aufnahme und Darstellung von Zähnen bzw. deren Präparationen und deren unmittelbare Nachbarschaft mittels einer Kamera für die Gewinnung von dreidimensionalen Daten zur Herstellung eines Passkörpers sowie eine Vorrichtung zur Durchführung dieses Verfahrens. Solche Verfahren finden in der folgenden Situation Anwendung:

Der Zahnarzt präpariert einen kariösen Zahn derart, dass sich dieser mittels einer Einlagefüllung (inlay, onlay) oder einer Krone in seiner Form und Funktion wiederherstellen lässt. Nachdem die entsprechende Präparation am befallenen Zahn vorgenommen ist, stellt sich das Problem der exakten, dreidimensionalen Vermessung der Präparation einschliesslich ihrer unmittelbaren Umgebung.

Nach vollzogener Vermessung wird die Aufnahme am Bildschirm ergänzt, um schliesslich mittels einer numerisch gesteuerten Werkzeugmaschine die gewünschte Rekonstruktion zu fertigen.

In den letzten Jahren wurden eine ganze Anzahl solcher Verfahren veröffentlicht. Im folgenden seien die wesentlichen kurz beschrieben:

Zuerst erwähnt wurde die Idee der Vermessung einer Zahnpräparation und deren Ergänzung in einem Bericht von B. Altschuler im November 71. Im darauf folgenden Report "An Introduction to Dental Laser Holography", USAF report SAM-TR-73-4 AD-758 191 (März 1973) findet sich auf Seite 10 folgender Gedankengang: "Mittels Laser Holographie gewonnene Zahn-Daten können eine Werkzeugmaschine steuern, um aus einem Goldblöckchen die inneren und äusseren Konturen einer passenden Krone zu fräsen".

Im Anschluss an dieses Dokument findet sich ein weiterer Bericht desselben Autors in "Laser Holography in Dentistry", Journal of Prosthetic Dentistry, vol 38, Nr. 4 aug.77 pp 216-225. Darin wird auf die Inlayversorgung hingewiesen und zugleich erwähnt, dass Prototypen eines solchen Apparates (Laser Holograph, Stereokomparator und NC Maschine) an der Brooks Air Force School of Medicine erprobt würden. Die praktisch gleiche Methode wurde etliche Jahre später von Dr. F. Duret in Europa zum Patent angemeldet (EP 0 040 165). Entsprechend dem fortgeschrittenen Stand der Technik gesellte sich dabei zum klassischen Hologramm noch die Methode der direkten interferometrischen Erfassung mittels Bildsensor, sowie die anschliessende, vollautomatische Formergänzung anhand von "a priori" Kenntnis bezüglich der Zahnmorphologie.

Eine weitere, frühe Beschreibung einer Rekonstruktionsmethode findet sich im US Patent von W.E. Swinson (US Pat. 3 861 044). Darin wird die optische Vermessung mit einer gewöhnlichen Kamera beschrieben - ein Verfahren, bei dem vieles unklar bleibt und das in der dargestellten Weise kaum durchführbar sein dürfte.

In einem Artikel von E.D. Rekow und A.G. Erdman, "Computer-Aided Automatic Production of Dental Restorations" Proceedings of the 4th Meeting of the urop. Soc. of Biomechanics Davos, Swiss Research Institute, p. 117ff, sind weitere Verfahren zur Gewinnung des "optischen Abdrucks" aufgelistet, insbesondere die stereometrische Auswertung eines Bildpaares, wie sie in der kartographischen Photogrammetrie allgemein Verwendung findet. Der Zahnarzt photographiert den präparierten Zahn mit einer Stereokamera und sendet anschliessend den Film oder das Sofortbild in ein zentrales Labor. Dort findet die Auswertung statt, die dann zur Steuerung der Maschine (ebenfalls im zentralen Labor) benützt wird.

Ein weiteres Verfahren beschreibt Dr. P. Heitlinger in der deutschen Offenlegungsschrift DE 29 36 847 Al. Bei dieser Methode wird über ein zahntechnisches Arbeitsmodell der Zahnstumpf stereoptisch erfasst, mittels Rechner die stereoptische Aufnahme ausgewertet und dann in bekannter Weise weiterverarbeitet.

Schliesslich sei auf die veröffentlichten Patentschriften der Anmelder selbst hingewiesen, nämlich EP 0 054 785, sowie US Pat. 4 575 805. Die darin beschriebene Methode der Erfassung der Zahnpräparation durch aufprojiziertes Muster und Triangulation, und die interaktive Ergänzung der registrierten Daten durch den Zahnarzt am Bildschirm, liefern die beste und rascheste Grundlage zur Fertigung der Zahnrekonstruktion mit einer numerisch gesteuerten Werkzeugmaschine.

Aus den zitierten Veröffentlichungen ergibt sich zusammenfassend etwa folgender Arbeitsablauf:

1. Der Zahnarzt nimmt, direkt in der Mundhöhle, eine räumliche Vermessung des präparierten Zahnes vor.

2. Die registrierte Form der Präparation kann auf zweierlei Arten dargestellt werden - als helligkeitscodiertes Relief - oder auf perspektivische Weise. Anhand einer dieser Darstellungen wird die Aufnahme kontrolliert und gegebenenfalls neu aufgenommen.

3. Anhand der dreidimensionalen Darstellung wird eine manuelle oder automatische Formergänzung vorgenommen, mittels der sich auf einer Maschine der gewünschte Passkörper aus dem Vollen schliefen lässt.

Dabei stellen sich die folgenden, bisher ungelösten Probleme, die eine praxisgerechte, d.h. zeit- und kostensparende Durchführung dieses Ablaufs behinderten. Die räumliche Vermessung kann nur unter bestimmten Bedingungen, insbesondere bei korrekt auf den präparierten Zahn ausgerichteter Kamera erfolgen. Die Einhaltung dieser Bedingungen ist bei den bekannten Methoden erst nachträglich, d.h. nach der Aufnahme und deren Auswertung verifizierbar.

Dies ist im besten Fall mit langer Wartezeit verbunden oder es können gar mehreren Sitzungen nötig werden. Der Zahnarzt verfügt damit bei der räumlichen Vermessung des präparierten Zahns über kein direktes "feed-back", was diesen Vorgang wesentlich erschwert. Liegt dann das Vermessungsresultat korrekt vor, so erfolgt gemäss dem oben Gesagten die Formergänzung, um die Konturen des gewünschten Passkörpers festzulegen. Hierfür ist bisher ebenfalls keine geeignete Darstellungsmöglichkeit der aufgenommenen Zahnpräparation beschrieben worden. Insbesondere eignen sich hierfür abstrakte, dreidimensionale Darstellungen schlecht, weil sie stark von dem Bild abweichen, das sich für den Zahnarzt von der Präparation visuell ergibt.

Es stellt sich damit die Aufgabe, ein Verfahren der eingangs genannten Art so zu verbessern, dass es praxisgerecht und nicht nur unter Laborbedingungen einsetzbar ist und insbesondere die oben erwähnten Nachteile nicht aufweist.

Das erfindungsgemässe Verfahren ist nun dadurch gekennzeichnet, dass zur Bestimmung der geeigneten Aufnahmestellung der Kamera ein pseudoplastisches Video-Suchbild erzeugt wird und dass auf einen Auslösebefehl hin in der dem letzten Suchbild entsprechenden Stellung ein Standbild erzeugt wird, wobei die diesem Standbild entsprechenden Kontrast- und Tiefenwerte zu jedem Bildpunkt bestimmt und für die weitere Bildverarbeitung gespeichert werden.

Dies erlaubt es dem Zahnarzt, präparierte Zähne in der Mundhöhle mittels einer einzigen Aufnahme räumlich zu erfassen und darzustellen.

In einer Suchphase, in welcher ein ungestörtes Videobild auf einem Monitor darstellbar ist, wird die Mundkamera positioniert. Unmittelbar anschliessend kann die räumliche Vermessung durch Aufprojektion eines Referenzmusters und Triangulation erfolgen.

Neben der augenblicklichen Bestimmung des dreidimensionalen Reliefs wird zusätzlich ein pseudoplastisches Kontrastbild ermittelt, das ebenfalls auf dem Monitor darstellbar ist.

Die Zuordnung Kontrastbild/Tiefenwerte erlaubt die manuelle Formergänzung am Kontrastbild, wonach anhand der gespeicherten Tiefendaten die räumliche Form in einem Rechner aufgebaut werden kann. Die rechnergestützte Bildverarbeitung kann sich der Kontrastinformation bedienen, um die Tiefendaten durch den Kontrast zu gewichten. Damit gelingt es, das eingangs erwähnte Verfahren hinsichtlich zweier, wesentlicher Aspekte zu verbessern:

1. Um sicher zu stellen, dass die räumliche Aufnahme auch in der richtigen Lage geschieht, so dass alle kritischen Partien registriert werden, steht dem Zahnarzt vor der räumlichen Vermessung, sozusagen als Einstellhilfe, ein Suchbild von bestmöglicher Qualität zur Verfügung. Durch diese Verbesserung wird das Arbeiten mit einem räumlichen Vermessungssystem stark erleichtert. Das bewegte Suchbild und die dreidimensionale Vermessung werden zeitlich derart kurz hintereinander vorgenommen, dass die Kamera zur Vermessung in der eingestellten Lage gehalten werden kann. Nach der eigentlichen Vermessung erscheint ferner augenblicklich eine Kontrollanzeige, so dass dem Zahnarzt der notwendige "feedback" unverzüglich geliefert wird.

2. Damit sich die räumliche Aufnahme auch visuell leicht interpretieren lässt, ist die entsprechende Standbild-Darstellung der Präparation ähnlich kodiert, wie das Suchbild. Beide Male erscheint auf dem zweidimensionalen Schirm ein intensitätsmoduliertes Bild. Dieses Bild ist pseudoplastisch. Der pseudoplastische Charakter kommt dadurch zustande, dass das menschliche Gehirn gewöhnt ist, zweidimensionale Bilder anhand ihrer Schattierung als räumliche Objekte zu interpretieren. Die Schattierung kann als zusätzliche Dimension der Gestalt der Präparation betrachtet werden. Die Berücksichtigun, dieser Information ist wesentliches Merkmal der vorliegenden Erfindung. Das Standbild kann damit als Zeichenebene für die Konstruktion des Passkörpers durch den Zahnarzt dienen.

Im nun folgenden Teil wird ein Ausführungsbeispiel der Erfindung anhand der Zeichnungen näher erläutert. Darin zeigen:

Figur 1 ein Flussdiagramm des verfahrensmässigen Ablaufs der Registrierung und Darstellung,

Figur 2 die wesentlichen Komponenten der Aufnahmekamera,

Figur 3a den Intensitätsverlauf eines Ausschnittes einer Bildsensorzeile während der ersten Aufnahmephase,

Figur 3b die Stellung, bei der das Referenzgitter um 180° verschoben wurde,

Figur 3c die Differenz zweier Teilbilder mit jeweils supplementärer Phasenlage,

Figur 3d die Differenz aus den Teilbildern mit dem Referenzmuster in der 90° resp. 270° Phasenlage,

Figur 4 ein Flüssigkristallelement, mit der dazugehörigen Ansteuerung, welches sich zur Erzeugung der verschiedenen Phasen des Referenzgitters besonders eignet,

Figur 5 ein Blockdiagramm der verwendeten Schaltung, die eine schnelle Transformation der aufgenommenen Teilbilder ermöglicht. Die zur Wandlung benützten Festwertspeicher sind im Detail hervorgehoben.

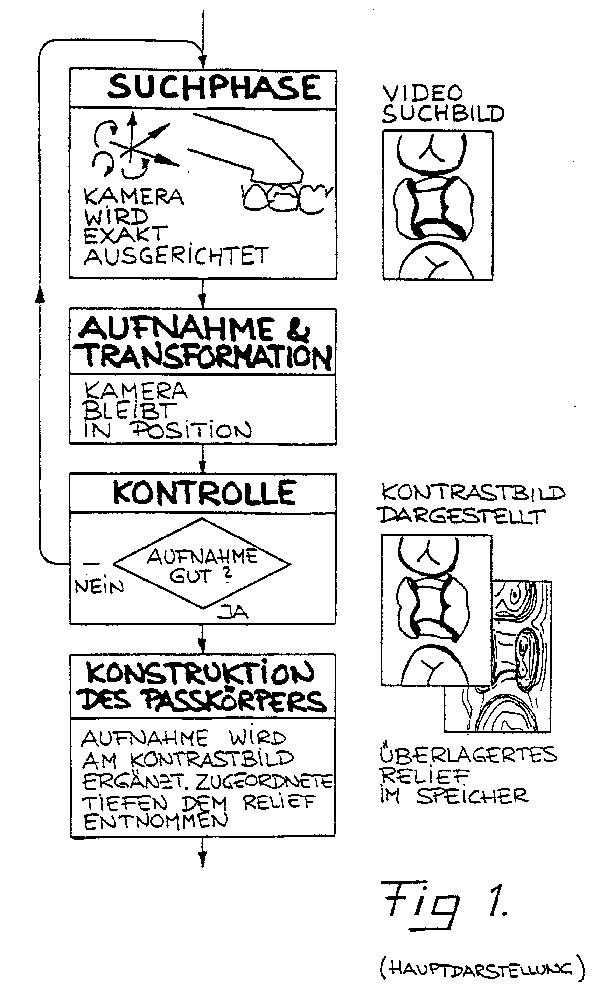

Bevor auf die einzelnen Aspekte im Detail eingegangen wird, soll zunächst anhand von Fig. 1 der Aufnahme- und Vermessungsvorgang übersichtsartig dargestellt werden.

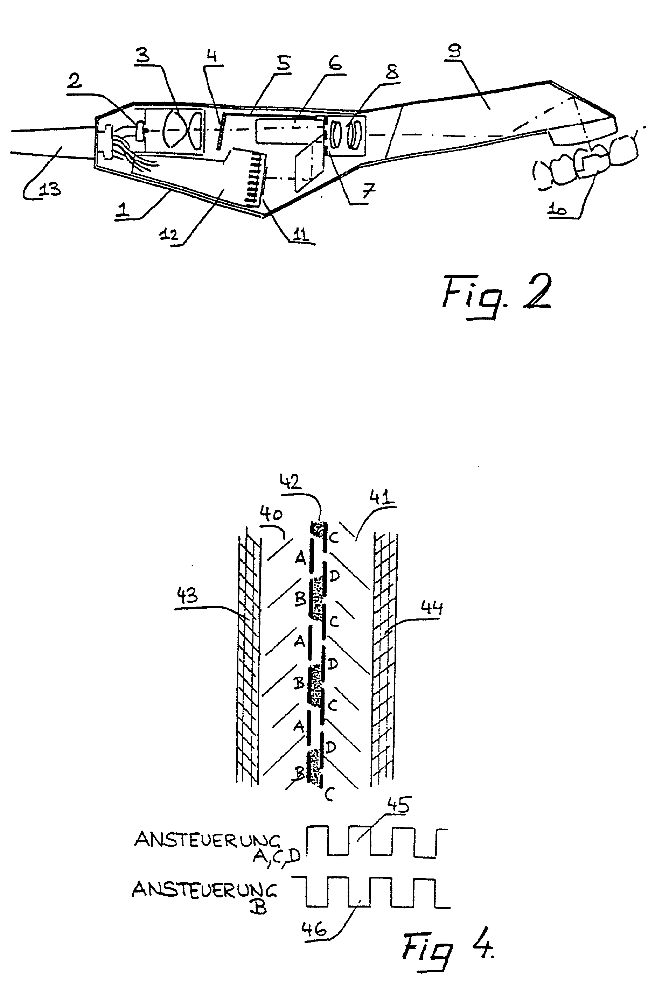

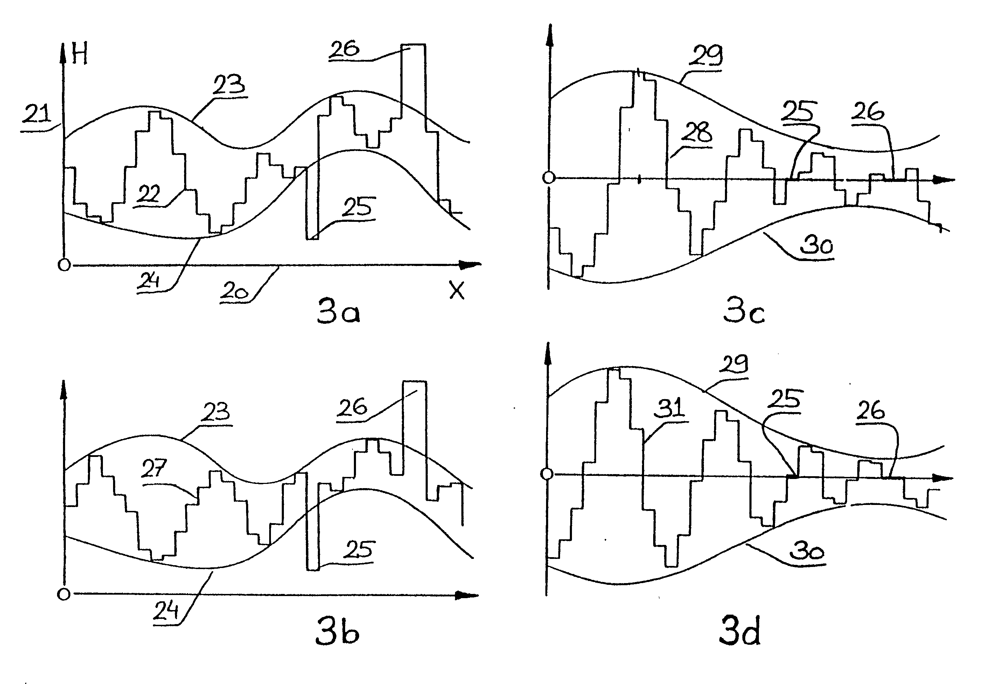

Während einer dem Vermessungsvorgang vorangehenden Suchphase arbeitet das System zunächst im wesentlichen wie ein herkömmliches Videosystem. Die Zahnpartie, welche im Aufnahmefenster erscheint; wird durch Abbildungsoptik und Sensor registriert und kann mit einer Bildfrequenz von z.B. 50Hz zur Darstellung gebracht werden, womit sich ein Standard-Fernsehbild ergibt. Der beobachtete Zahn wird alleidings durch die Kamera zum Zweck der räumlichen Vermessung beleuchtet und zwar mit einem gitterartigen Referenzmuster. Dieses Referenzmuster sollte jedoch nicht auch auf dem Suchbild erscheinen. Die erste Aufgabe besteht darin, das dem eigentlichen Bildinhalt überlagerte und störende Muster zu eliminieren. Wie das geschehen kann wird weiter unten, anhand der Figuren 3 und 4 erläutert.

Die Suchphase dient zur Auffindung der optimalen Bedingungen für die anschliessend erfolgende, dreidimensionale Aufnahme. Dabei gilt es zu beachten, dass die Belichtung richtig gewählt wird, d.h. die Dynamik des Sensors voll ausgesteuert wird. Eine genaue Passung der Rekonstruktion ist nur dann gewährleistet, wenn bereits bei der Aufnahme alle kritischen Partien abgebildet werden. Die Positionierung der Kamera in der Suchphase muss also so gewählt werden, dass sie mit der späteren Einschubachse des Passkörpers (Inlay etc.) übereinstimmt. Schliesslich muss der Abstand Kamera zu Zahn so eingestellt werden, dass dieser gesamthaft scharf abgebildet wird. Alle diese Einstellungen nimmt der Zahnarzt in der Suchphase direkt am Patienten vor, ohne ihn durch zusätzliche Vorrichtungen in einer starren Lage zu fixieren - die Kamera soll von Hand in die Aufnahmelage gebracht werden. Um das Ausrichten der Kamera in allen räumlichen Freiheitsgraden zu erleichtern, sind im Video-Suchbild Marken eingeblendet (Fenster, Fadenkreuz, mm-Teilung etc.).

Ist die gewünschte Einstellung gefunden, löst der Zahnarzt, z.B, durch ein Pedal, den eigentlichen Vermessungsvorgang aus.

Während dem Vermessungsvorgang wird das Referenzmuster in verschiedenen räumlichen Lagen auf die Zahnoberfläche projiziert. Die dabei entstehenden Bilder werden in einem Speicher festgehalten. Dies dauert insgesamt weniger als 1/5 Sekunde. Danach wird die abgespeicherte Information transformiert; auch dieser Schritt dauert weniger als 1/5 Sekunde.

Als Ergebnis der Transformation befinden sich im Speicher neue Daten, nämlich das Relief der Präparation (vgl. US Pat. 4 575 805) in Form von Tiefendaten zu jedem Bildpunkt und zusätzlich ein Kontrastbild das unmittelbar zur Darstellung gelangen kann. Dieses Bild gleicht in seiner pseudo plastischen Art dem Video-Suchbild und gestattet dadurch dem Zahnarzt eine sofortige Kontrolle der Aufnahme. Das Kontrastbild erscheint dem Zahnarzt als Standbild des letzten Video-Suchbilds, bei welchem er kurz zuvor den Vermessungsvorgang ausgelöst hat.

Anhand dieses Standbilds kann der Zahnarzt sich vergewissern, ob die Aufnahme eine geeignete Grundlage für die nachfolgende Konstruktion des Passkörpers bildet. Wenn nicht, kann erneut eine Suchphase eingeleitet werden.

Heisst er jedoch die Aufnahme gut, so sind damit bereits auch sämtliche Daten von der räumlichen Gestalt der Zahnpräparation im Speicher vorhanden und es kann unverzüglich mit der Konstruktion des Passkörpers begonnen werden. Für diesen interaktiven Verfahrensschritt dient das erwähnte Kontrastbild als Konstruktions-Grundlage. Das Kontrastbild ist, wie die Erfahrung gezeigt hat, für den Zahnarzt weit besser interpretierbar als irgendeine abstrakte, nur auf Tiefendaten beruhende Darstellungsweise. Im Kontrastbild sind auch feine Linien enthalten, die Risse, Kanten und andere Merkmale charakterisieren. Anhand dieser zusätzlichen Dimension , die diese wesentlichen Merkmale vermitteln, kann der Zahnarzt beispielsweise eine auf der Höhe des Zahnfleisches verlaufende Präparationsgrenze sicher identifizieren, welche sich anhand der Reliefdarstellung allein nicht erkennen liesse.

Der Speicher weist wie noch näher dargelegt wird, zwei überlagerte Speicherebenen "A" und "B" auf. Diese sind so organisiert, dass in der Ebene "A" das Kontrastbild liegt, welches zur Anzeige gelangt. In der für den Zahnarzt unsichtbaren Ebene "B" sind zu jedem Bildpunkt die zugehörigen Tiefenwerte (Z-koordinaten) gespeichert. Während der Zahnarzt ausschliesslich anhand des ihm vertrauten, pseudoplastischen Kontrast-Bildes graphisch rekonstruktive Eingaben erarbeitet, entsteht in der Ebene "B" eine dreidimensionale Form, die aus Tiefendaten und den anhand der in Ebene "A" eingegebenen Grenzlinien besteht und die Grundlage für die automatische Herstellung eines entsprechenden Passkörper bildet. Die Ebene "A" ist zu diesem Zweck mit einer zusätzlichen "overlay" Ebene ausgerüstet, welche die mittels Zeichenhilfe (Maus, Trackball etc.) eingegebenen Grenzlinien darstellt und auch speichert.

Ist auch die Konstruktionsphase abgeschlossen, so liegen im Speicher alle Daten vor, welche die mittels Zeichenhilfe (Maus, Trackball etc.) eingegebenen Grenzlinien darstellt und auch speichert.

Ist auch die Konstruktionsphase abgeschlossen, so liegen im Speicher alle Daten vor, welche die Geometrie des Passkörpers bestimmen. Diese Daten werden zur Steuerung einer NC-Bearbeitungsmaschine verwendet, welche den Passkörper in wenigen Minuten aus dem Vollen schleift (vgl. EU-A-0182 098).

Nach dieser Uebersicht über den Verfahrensablauf soll nun zunächst anhand von Fig. 2 Aufbau und Funktion der Mundkamera näher erläutert werden. Die Kamera besitzt ein Gehäuse 1, das mit seiner äusseren Formgebung das Einführen der Kamera in den Mund des Patienten gestattet. Im Gehäuse ist eine Lichtquelle 2 untergebracht, vorzugsweise eine Licht emittierende Diode LED, welche im nahen Infrarotbereich arbeitet. Durch die LED wird über ein Kondensorglied 3 eine Maske 4 beleuchtet. Diese Maske kann entweder als klassisches Gitter, welches mechanisch bewegt wird ausgeführt sein. In diesem Fall ist die Maske 4 an einem Biegeelement 5 befestigt, mittels welchem sie senkrecht zum Strahlengang bewegt werden kann. Sie kann aber auch als Flüssigkristallzelle ausgeführt sein, welche ohne bewegte Teile auskommt wie anhand der Fig. 4 noch erläutert wird. In praktisch der gleichen Weise wie beim Diaprojektor wird das Referenzmuster so über ein Objektiv 8 auf die Zahnreihe 10 projiziert. Zur Trennung des Beleuchtungskanals und des Beobachtungskanals ist im Strahlengang eine Zweilochblende 7 und ein Strahlenteiler 6 vorgesehen, welche das von der Zahnreihe 10 zurückgestreute Licht geometrisch vom Beleuchtungskanal trennen und den Parallaxwinkel definieren, der für das räumliche Triangulationsverfahren ausschlaggebend ist. Ueber ein langgezogenes Prisma 9 mit angekitteter Feldlinse entsteht ein telezentrischer Strahlengang. Ein Bildsensor 11 empfängt das von den Zähnen zurückgestreute Licht und wandelt dieses in ein zeilengerastertes Videosignal. Die Ansteuer- und Anpassnetzwerke, sofern nicht ausserhalb der Kamera, sind auf einem Print 12 untergebracht. Die Kamera ist durch ein mehradriges, elektrisches Kabel 13 mit der Rechner- und Darstellungseinheit verbunden.

Anhand der Figuren 3a bis 3d wird nun das Verfahren der Suchbilddarstellung sowie der Tiefenmessung mit gleichzeitiger Kontrastbestimmung mittels Projektion des Referenzmusters erläutert.

Grundsätzlich eignen sich die verschiedensten Referenzmuster zur Durchführung des Vermessungsverfahrens. Im vorliegenden Ausführungsbeispiel wird von einem Streifenmuster ausgegangen, bei welchem die Streifen jeweils zur Hälfte schwarz (lichtundyrchlässig) und zur Hälfte weiss (lichtdurchlässig) ausgebildet sind. Das Referenzmuster ist ferner periodisch. Die sog. Gitterperiode definiert, analog zu zeitlichen Signalen, das Intervall von einem Uebergang von schwarz auf weiss zum nächsten solchen Uebergang als 360°. Innerhalb einer solchen Periode kann dann sinngemäss von einer Phase geredet werden. So ist z.B. die Stelle des Uebergangs von weiss auf schwarz als 180° gekennzeichnet.

Wird dieses Referenzmuster, das zunächst einen rechteckigen Helligkeitsverlauf erzeugt, über das Abbildungsobjektiv auf die Zahnreihe geworfen und das entstandene Bild wieder durch das Abbildungsobjektiv auf dem Sensor abgebildet (vgl. Fig 2), so findet ein erheblicher Schärfenverlust statt. Dieser bewirkt, dass die Helligkeitskurve beim Empfang praktisch sinusförmigen Verlauf hat. Anhand dieser sinusförmigen Signale, die vom Bildsensor 11 registriert und gewandelt werden, betrachten wir nun die verschiedenen Aufnahmestadien. Angenommen, die Maske 4, welche das Referenzmuster erzeugt, steht fest im Raum, so detektiert der Sensor 11 die Helligkeitswerte gemäss Figur 3a. Die horizontale Achse 20 entspricht einem Ausschnitt aus einer Zeile des Bildsensors, bestehend aus mehreren Bildpunkten. Für jeden Bildpunkt x wird ein Wert H detektiert, der grundsätzlich der vom Zahn zurückgestreuten Helligkeit entspricht. In der Praxis treten aber eine Reihe von zusätzlichen Einflüssen auf, die die theoretischen Werte verfälschen und letztlich das Messresultat generell verschlechtern. Mit dem vorliegenden Verfahren gelingt es, diese Messfehler zu erkennen, zu klassieren und zu eliminieren, wie nachfolgend gezeigt wird.

Verbindet man die entlang einer Zeile von Bildpunkten gewonnenen Helligkeitswerte, so entsteht eine Kurve 22. Diese Kurve wird durch mehrere Faktoren moduliert. An erster Stelle erkennt man den durch das Streifenmuster erzeuten sinusförmigen Verlauf. Wie bereits bekannt (US Pat 4 575 805), liegt in der Phase die Tiefeninformation. Die Auswertung dieser Tiefeninformation wird weiter hinten erläutert. Soll nun zunächst ein ungestörtes Video-Suchbild erzeugt werden, so stört diese Modulation, welche im Suchbild als Streifenmuster erscheinen würde. Bildet man in Fig 3a eine Enveloppe 23, die jeweils die Maxima der Kurve 22 verbindet, so ergibt diese Kurve den ungestörten Helligkeitsverlauf. Zur Erzeugung des ungestörten Suchbilds ist also die durch das projizierte Streifenmuster erzeugte Modulation zu entfernen, so dass nur die Enveloppe 23 verbleibt. Dies ist auf zwei Arten möglich:

Einerseits kann die Maske 4 aus dem Strahlengang entfernt werden, was in der Publikation "Marginale Adaptation von adhäsiven Porzellaninlays in vitro" (Schweiz. Mschr. Zahnmed. 95 1118 1985) durch physikalisches Entfernen der Gitterplatte geschah. Wird gemäss Fig 4 eine Flüssigkristallzelle als Maske eingesetzt, so erfolgt die Wegnahme auf elektronische Weise. Figur 4 zeigt eine Schnittdarstellung einer entsprechenden Flüssigkristallzelle. Zwischen zwei Glasplatten 40,41 wird in bekannter Weise ein Hohlraum gebildet, der mit einer ebenfalls bekannten Flüssigkeit 42 gefüllt wird. Weitere Komponenten, die aus der herkömmlichen Technologie stammen, sind die beidseitig aufgeklebten Polarisationsfilter 43,44. Entsprechend der verwendeten Lichtquelle, die im nahen Infrarotbereich arbeitet, sind sowohl die Flüssigkeit als auch die Filter für diese Wellenlänge optimiert. Besonders an der beschriebenen Vorrichtung ist nun die Anordnung der Elektroden A,B,C und D beidseits des Hohlraums. Während auf der linken Seite die Elektroden A und B alternieren (A und B sind jeweils untereinander elektrisch verbunden), sind auf der rechten Seite C und D analog angeordnet, wobei die linken und rechten Paare zueinander um eine halbe Elektrodenbreite verschoben sind, wie aus der Abbildung ersichtlich ist. An die insgesamt vier, nach aussen geführten Kontaktstellen werden nun Rechtecksignale 45 resp. 46 angelegt, welche gestatten, die zur Aufnehmesequenz gehörenden Phasenverschiebungen in Inkrementen von 90° zu erzeugen. Im gezeichneten Fall sind alle B Elektroden mit dem Signal 45 angeregt, die Elektroden A,C und D mit dem Signal 46. Wo sich gegenphasig angeregte Elektroden gegenüberstehen, richten sich im entstehenden Spannungsfeld die Kristalle so aus, dass mit den Filtern 43,44 kombiniert, eine Sperrung des Lichtes erfolgt. Die im Hohlraum dunkel gezeichneten Stellen sollen diesen Effekt veranschaulichen. Einander gegenüberstehende Elektroden, gleicher Anregung belassen die Flüssigkristalle in Ruhelage, d.h. das Licht tritt ungemindert durch Zyklisches Vertauschen der Ansteuerung (immer eine Elektrodengruppe in Gegenphase zu den andern drei)gestattet es, das Gittermuster in der gewünschten Weise zu verschieben, ohne dass dabei mechanische Bewegungen auftreten. Diese Lösung garantiert eine präzise räumliche Phase des Referenzmusters. Ausserdem wird durch die Flüssigkristallzelle, welche fest in die Kamera eingebaut werden kann, die gesamte Kamera unempfindlicher auf Erschütterungen.

Auch der Suchphase wird auf elegante Weise Rechnung getragen. Wenn alle Elektroden mit der selben Phase angeregt werden, oder allgemein, sich auf dem selben Potential befinden, ist das Flüssigkristallelement strukturlos, wirft also keine störenden Streifen auf die Präparation.

Die zweite Art, das Referenzmuster zu eliminieren, besteht im "Verwischen" desselben. Der Bildsensor 11 arbeitet ja als Integrator, d.h. die auftreffenden Lichtquanten werden über eine gewisse Zeit (40ms) aufsummiert, und die derart gewonnene Ladung durch einen Schiebebefehl in ein Ausleseregister transferiert. Wird das Streifenmuster mittels dem Biegeelement 5 derart bewegt, dass pro Integrationsperiode alle Punkte der Zahnoberfläche gleich viel Licht erhalten, so verschwindet das störende Muster durch Integration. In der Praxis empfiehlt es sich, das Gitter mit einer sinusförmigen Ansteuerung auszulenken, deren Amplitude $\sqrt{2}$ mal der Periode des Referenzmusters entspricht. Das so erzeugte Bild wird als ungestörtes Video-Suchbild in der Suchphase verwendet.

Nachdem die Erzeugung des Suchbilds geklärt ist, soll nun die Erzeugung des Kontrastbilds sowie des zugehörigen Tiefenwerts (Z-Koordinate) erläutert werden. Neben der Enveloppe 23 ist hierfür auch die Kurve 24 in Fig 3a zu beachten. Sie vereinigt Streulicht (nicht direkt vom Zahn zurückgeworfen) und den Dunkelstrom des Sensors. Dieser Falschlichtanteil 24 ist zunächst zu eliminieren, da er für die Messung keinerlei nützliche Information beinhaltet. Die Differenz zwischen der oberen Enveloppe 23 und der Kurve 24, sozusagen der unteren Enveloppe, ergibt für jeden Bildpunkt x den Kontrast des aufprojizierten Musters. Die Erzeugung eines solchen Kontrastbildes erlaubt es, Fehler im Bildsensor 11 sowie störende Lichtreflexe auf Zähnen und Zahnfleisch zu eliminieren.

Die Stelle 25 soll z.B. den Fall eines "blinden" Bildpunkts des Sensors 11 veranschaulichen. Durch Verunreinigungen, sowie die engen Toleranzanforderungen bei der Halbleiterfertigung kommt es häufig vor, dass einzelne Elemente (Bildpunkte) aber auch Gruppen bis ganze Spalten unkontrollierbare Werte abgeben. Sensoren, die frei von solchen Fehlern sind, sind in der Regel sehr kostspielig. An der Stelle 26 befindet sich andererseits eine Glanzstelle (Reflex) des Zahns oder Zahnfleisches, welche den Sensor übersteuert. Dies kann so störend ausfallen, dass der Zahnarzt schon in der Suchphase das Problem erkennt und z.B. durch Mattieren beseitigt. Die Stelle kann aber auch örtlich klein sein und doch die Messwerte beeinflussen, weil die Dynamik des Sensors oder des nachfolgenden Analog/Digitalwandlers nicht ausreicht. Anhand der Fig 3b und 3c wird nun erläutert, auf welche Weise durch Erzeugung eines Kontrastbilds solche Fehler eliminiert werden können.

Verfolgen wir zunächst in Fig 3b, wie die Aufnahmesequenz weiterläuft: Lassen wir das Referenzmuster sich soweit verschieben, dass wo im Bild 3a die hellen Partien jetzt die dunklen liegen und umgekehrt, was einer Verschiebung von 180° also einer halben Periode entspricht. Dann zeigt sich folgende Situation (die gleiche Stelle auf der Zahnoberfläche sei bei gleicher Beleuchtung abgebildet): Die Helligkeitskurve 27 hat sich bezüglich der Ausgangslage ebenfalls um 180° verschoben. Die Enveloppe 23 und der Falschlichtanteil 24 sind jedoch gleich geblieben. Auch die Zonen des blinden Sensorelements und des Reflexes 25,26 sind im wesentlichen unverändert. Eine Subtraktion der beiden Signale von Fig 3a und Fig 3b eliminiert demzufolge den Anteil 24 vollständig. Die Subtraktion an den Stellen 25,26 ergibt beide Male den Wert null.

Figur 3c zeigt das so gebildete Differenzsignal 28 entlang dem betrachteten Abschnitt, wie es nun im Speicher steht. Der Verlauf des Differenzsignals 28 gleicht der ursprünglichen Kurve 22, wobei sich die Amplitude verdoppelt hat und das Signal bipolar geworden ist, d.h. es bewegt sich beidseits der Nullinie. Die durch Subtraktion gewonnene Kurve 28 lässt sich ebenfalls durch eine Enveloppe umfassen. Im positiven Bereich ist dies die Kurve 29, im negativen die Kurve 30, welche symmetrisch zu 29 verläuft. Diese Kurven beschreiben nicht wie in Figur 3a die lokale Helligkeit, sondern den Kontrast. Der Kontrast bezeichnet die Differenz zwischen der an jedem Punkt erscheinenden Helligkeit bei maximaler Beleuchtung (Referenzmuster dürchlässig) und der minimalen Beleuchtung (Referenzmuster undurchlässig). Während das Helligkeitssignal des Video-Suchbildes durch einen erheblichen Anteil Störinformation verfälscht ist, beinhaltet das Kontrastsignal alle zur Tiefenmessung relevanten Daten.

Anhand von Figur 3d soll nun noch gezeigt werden, wie aus der Situation gemäss Fig 3c und Fig 3d schliesslich die geforderte Tiefenmessung und die Bestimmung des Kontrasts abgeleitet wird.

Macht man in analoger Weise zu den in den Figuren 3a und 3b gezeiten Lagen, je eine Aufnahme mit der Phase 90° und 270° und subtrahiert die beiden voneinander, so entsteht der in Figur 3d skizzierte Verlauf. Umhüllt von den selben Enveloppen 29,30 erscheint das bipolare Signal 31, welches gegenüber dem Differenzsignal 28 einen Gangunterschied von 90° aufweist. Betrachten wir diese zwei Signale als den Real- und Imaginärteil der komplexen Ortsfrequenz, wie dies im US Pat 4 575 805 beschrieben wird. Durch die Operation:

$$\theta = \arctan \frac{Im}{Re} \text{ /: sign ( Im ) wird}$$

aus dem Wertepaar, das durch die zwei Signale zu jedem Bildpunkt x gebildet wird, eine "Phase" errechnet. Diese "Phase" ist ein Mass für die Tiefenlage jedes Bildpunkts x. Zusätzlich kann aus demselben Wertepaar mittels der Funktion

$$K = \sqrt{Re^2 + Im^2}$$

nun aber auch der Kontrast für jeden Bildpunkt ermittelt werden, der im wesentlichen der in Fig 3c und 3d dargestellten Enveloppe 29 entspricht. Durch diese Erweiterung erhält man mittels der Kontrastbestimmung eine Aussage über die Gewichtung eines jeden Bildpunktes, d.h. ein Gütekriterium betreffend die errechnete Tiefe. Ferner gestattet die Kontrastbestimmung ohne Einbusse an Auflösung (Verschmieren des Bildes) eine Darstellung der geforderten, feingliedrigen, zur Beurteilung unerlässlichen Details.

Zur Gewichtung: Da das System mit quantisierten Daten arbeitet, ist es einleuchtend, dass mit der Verkleinerung des Kontrasts (steile Wände, Schattenpartien) die Quantisierung immer gröber wird und die errechnete Tiefe mit stets schlechterer Auflösung ermittelt werden kann. Anderseits grenzen an alle dunklen (kontrastarmen) Partien auch wieder hellere an, so dass eine Interpolation im Bereich der dunklen Zonen die Daten statistisch verbessern kann. Nehmen wir an, dass alle Tiefenwerte mit der selben Gewichtung berücksichtigt würden, wie dies der Fall wäre, wenn die Kontrastinformation fehlte, so könnte die besagte Datenverbesserung nicht vorgenommen werden. Die Gewichtung erlaubt es, auch die bereits erläuterten Störeinflüsse zu berücksichtigen, was anhand des blinden Elementes und des Reflexes erläutert sei: Wie wir gesehen haben, ergibt die Subtraktion in beiden Fällen null, und zwar sowohl für den Realteil 28, als auch für den Imaginärteil 31. Offensichtlich ergibt die Auswertung an dieser Stelle keine sinnvollen Resultate. Neu ist aber die Möglichkeit durch Gewichtung, in diesem Falle Kontrast gleich null, dieser Situation auf elegante Weise Rechnung zu tragen. Als einfachste Möglichkeit sei auf die Schwellendiskrimination hingewiesen. Alle Punkte, die einen Kontrast bestimmter Grösse nicht überschreiten, werden als "Ausreisser" taxiert und von der Weiterverarbeitung ausgeschlossen. Dieses einfache Beispiel demonstriert die Leistungsfähigkeit der kombinierten Tiefen-/Kontrastbestimmung.

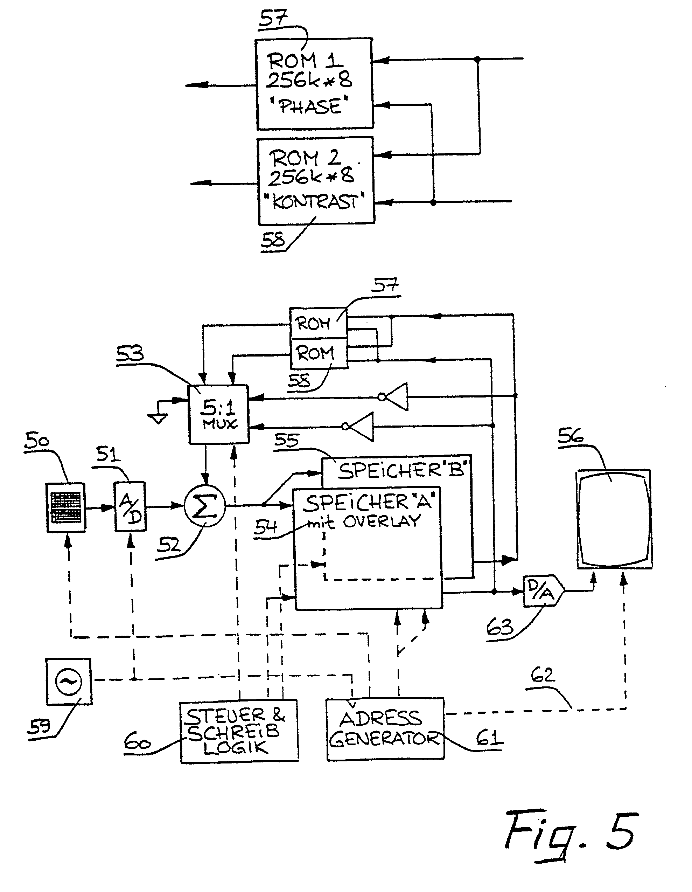

Das zur Durchführung der anhand der Figur 3 gezeigten Schritte erforderliche Speicher- und Rechenwerk ist in der Figur 5 abgebildet. Dabei wird zur Vereinfachung auf die Darstellung der äusseren Zu- und Wegleitungen verzichtet. Diese dienen dem Zugriff des Prozessors (der zentralen Recheneinheit) auf jeden Punkt der Speicherebenen "A" und "B".

Die diagrammatisch dargestellte Anordnung bietet folgende Betriebszustände: Suchphase, Aufnahme eines Helligkeitsbildes ("A" oder "B"), Aufnahme mit gleichzeitiger Differenzbildung ("A" oder "B") und Transformation zur Gewinnung von Teife und Kontrast.

In der Suchphase (Referenzmuster synchron bewegt oder abgeschaltet) gelangt das Helligkeitssignal vom Bildsensor 50 über einen Analog/Digital Wandler 51 zu einem Addierer 52. Auf dem zweiten Eingang des Addierers erscheint der durch einen Multiplexer 53 angewählte Kanal, in diesem Betriebszustand null, wie durch das Erdsymbol angedeutet. Das somit unveränderte Signal wird mittels einer Schreibelogik 60 und einem Adressgenerator 61 in die Bildene "A" 54 abgespeichert. Der Adressgenerator erzeugt horizontale und vertikale Adresssignale, die die entsprechenden Speicherplätze ansteuern. Mittels ähnlichen Signalen wird auch der Bildsensor 50 ausgelesen. Gleichzeitig wird ein Synchronisationssignal 62 generiert, das zu einem Bildschrim 56 gelangt. Das laufend aus dem Speicher gelesene Signal läuft über einen D/A Wandler 63 zum Bildschirm, so dass eine laufende Anzeige des Speichers "A" geschieht. Ist diese Speicherebene zusätzlich mit einem "Overlay" ausgerüstet, welches vom Prozessor beschrieben werden kann, lassen sich dem Suchbild beliebige Marken (Fadenkreuz, mm Teilung etc. überlagern).

Neben den rein geometrischen Marken ist es auch möglich, Konturen von Zähnen, die in einer früheren Aufnahme vermessen wurden, einzublenden. Dadurch können zeitlich nacheinander liegende Aufnahmen räumlich zusammengefügt werden. Z.B. kann eine Krone an die kombinierte, ausgerichtete Aufnahme des Zahnstumpfs einerseits und einer Aufnahme der mit Wachs modellierten Okklusalpartie (Bissfläche) anderseits angepasst werden. In gleicher Weise können auch Brücken aus mehreren Gliedern, die nicht alle ins Aufnahmefenster passen, zur Konstruktion lagerichtig in mehreren Teilen aufgenommen werden. Durch dieses Ausrichten der Aufnahmen im Suchbild entfällt eine anschliessende, rechnerische Koordinatentransformation, die mit grossem Aufwand und Rundungsfehlern verbunden ist.

Wird nun vom Zahnarzt die Aufnahme ausgelöst, so geschieht folgendes: Das Referenzmuster wird in die erste Phasenlage 0° gebracht. Während eines Bildes liefert der A/D Wandler die Helligkeit (Kurve 22 Figur 3a). Diese wird, wie in der Suchphase, direkt in die Speicherebene "A" geschrieben. Für das nun folgende Bildintervall hat sich das Gitter in die 90° Lage verschoben, und die entsprechenden Helligkeitswerte gelangen in die Speicherebene "B". Der Multiplexer liefert immer noch den Ausgang null.

Beim dritten Bildintervall wird nun der Rückführungspfad geöffnet, womit der invertierte Ausgang der Ebene "A" am zweiten Eingang des Addierers erscheint. Auf diese Weise wird die Differenz 180° - 0° direkt in die Ebene "A" geschrieben. Dieser Vorgang wird als "read-modify-write" Operation beschrieben.

Hat das Gitter schliesslich die Phase 270° erreicht, so kann die Differenz 270° - 90° in die "B" Ebene geschrieben werden.

Die eigentliche Aufnahme ist nun fertig, die Lichtquelle, der Sensor und der A/D Wandler werden abgeschaltet. In der Speicherebene "A" befindet sich nun das Differenzsignal 28 der Phasen 0° und 180° (Fig 3c) und in der Speicherebene "B" das Differenzsignal 31 der Phasen 90° und 270° (Fig 3d), welche wie erläutert, als Imaginärteil Im und Realteil Re einer komplexen Zahl aufgefasst werden.

Nun erfolgt die Transformation dieser Daten zu den Kontrast- bzw. Tiefendaten K und θ. Während dieser Transformation wird zu jedem Bildpunkt aus dem gespeicherten Werkpaar Im, Re ein neues Datenpaar K,θ gebildet. In ROM-Speichern 57,58 sind hierfür die entsprechenden Funktionen:

$$\theta = \arctan \left| \frac{Im}{Re} \right| : sign\ (Im)$$
$$K = \sqrt{Re^2 + Im^2}$$

tabellarisch fest gespeichert

Der Adressgenerator liest nun abwechslungsweise einen Punkt aus der Ebene "A" und einen aus der Ebene "B", so dass an der ROM-Tabelleneinheit 57,58 immer ein komplexes Paar am Eingang steht. Beim Lesen des nächsten Punktes aus "A" steht ausgangs der Tabelle 58 der Kontrast, der im selben Zugriff in die soeben gelesene Speicherzelle geschrieben wird. Beim Lesen des Punktes aus der "B" Ebene sorgt der Multiplexer 53 dafür, dass diesmal die Tiefe (Phase) in die Zelle der Ebene "B" zurückgeschrieben wird. Da sich diese Transformation direkt im Speicher, und nicht über die zentrale Recheneinheit, durchführen lässt, steht das Kontrastbild nach ca. 100 Millisekunden im Speicher, wird somit augenblicklich am Monitor 56 dargestellt.

Damit steht das Kontrastbild als Standbild in der bereits erläuterten Art für die Konstruktion der Passkörperform durch den Zahnarzt zur Verfügung. Kongruent dazu sind die Tiefendaten abgespeichert, welche dem Rechner gestatten, die entsprechende Raumform des Passkörpers zu bestimmen und so die genannte Bearbeitungsmaschine anzusteuern.

Das beschriebene, erfindungsgemässe Vorgehen gestattet es, den an sich komplexen Aufnahme- und Vermessungsvorgang des präparierten Zahns zum Zweck der Konstruktion eines Passkörpers praxisgerecht auszuführen. Dem Zahnarzt liegen insbesondere nur solche Darstellungen als Grundlage für seine Entscheidungen und für die Konstruktion des Passkörpers vor, wie er sie aus herkömmlichen Zahnbehandlungen kennt und zu interpretieren versteht. Diese praxisnahe Darstellung lässt sich durch das erfindungsgemässe Vorgehen direkt und ohne zu grossen Rechenaufwand mit dem Vermessungsvorgang kombinieren. Der geringe Rechenaufwand ermöglicht einen Arbeitsablauf unter Echtzeitbedingungen und ohne Unterbrüche, welche sowohl für den Zahnarzt als auch den Patienten störend in Erscheinung treten würden. Unmittelbar an die Suchphase anschliessend und basierend auf der gleichen Ansicht der Zahnpräparation kann auf dem stehenden Kontrastbild in kurzer Zeit die Konstruktion des Passkörpers durch manuelle Formergänzung desselben vorgenommen werden. Die rechnergestützte Bildverarbeitung wertet die Kontrastinformation ebenfalls aus, indem die gespeicherten Tiefendaten durch den Kontrast gewichtet werden.

**Patentansprüche**

1. Verfahren zur räumlichen Aufnahme und Darstellung von Zähnen bzw. deren Präparationen und deren unmittelbarer Nachbarschaft mittels einer Kamera für die Gewinnung von dreidimensionalen Daten zur Herstellung eines Passkörpers, wobei zur Gewinnung der Tiefendaten ein Referenzmuster aufprojiziert wird, dadurch gekennzeichnet, dass zur Bestimmung der geeigneten Aufnahmestellung der Kamera zunächst ein bewegtes Video-Suchbild erzeugt und auf einem Monitor angezeigt wird, auf dem das aufprojizierte Referenzmuster nicht in Erscheinung tritt, und dass auf einen Auslösebefehl hin in Aufnahmestellung eine Bildaufnahmesequenz zur Erzeugung und Speicherung eines Datensatzes abläuft, der einerseits Bildinformation enthält, welche dem letzten Suchbild entspricht und in Form eines Kontrastbildes als Zeichenvorlage auf dem Monitor anzeigbar ist, und der andererseits zur Bildinformation deckungsgleiche Tiefenwerte enthält, so dass eine auf der Zeichenvorlage ausgeführte Konstruktion zusammen mit den Tiefwerten den Passkörper dreidimensional definiert.

2. Verfahren nach Anspruch 1, dadurch gekennzeichnet, dass als Standbild die Kontrastwerte, d.h. die um einen Falschlichtanteil korrigierten Helligkeitswerte, zu jedem Bildpunkt abgebildet werden, um eine pseudoplastische Darstellung der Präparation zu erzeugen.

3. Verfahren nach einem der vorangehenden Ansprüche, dadurch gekennzeichnet, dass im Videosuchbild bezüglich dem Bildrahmen feststehende Markierungen eingeblendet werden, welche gespeicherten Bilddaten entsprechen, so dass das Videosuchbild bzw. der aufzunehmende Datensatz auf die eingeblendeten Markierungen ausrichtbar ist.

4. Verfahren nach einem der vorangehenden Ansprüche, dadurch gekennzeichnet, dass das Standbild als Zeichenebene für die Konstruktion von Passkörperkonturen mit Hilfe von elektronischen Zeichenmitteln verwendet wird, wobei die eingegebenen Passkörperkonturen aufgrund der Zuordnung der Bildpunkte des Standbilds zu den entsprechenden Tiefenwerten zu dreidimensionalen Konturdaten erweitert werden.

5. Verfahren nach einem der vorangehenden Ansprüche 2 bis 4, dadurch gekennzeichnet, dass die Tiefenwerte mit Hilfe der zugehörigen Kontrastwerte für die Weiterverarbeitung gewichtet werden, so dass das statistische Gewicht jedes Tiefenwertes von der Grösse des entsprechenden Kontrastwertes abhängig ist.

6. Vorrichtung zur Durchführung des Verfahrens nach einem der vorangehenden Ansprüche, wobei ein Speicher- und Rechnerwerk (51 - 55, 57 - 63) vorgesehen ist, das aus den aufgenommenen Bildsignalen anhand des aufprojizierten verschieblichen Referenzmusters die dreidimensionalen Daten zur Herstellung des Passkörpers bestimmt, dadurch gekennzeichnet, dass zur Eliminierung des Musters für die referenzmusterfreie Darstellung des Videosuchbilds auf dem Monitor (56) Mittel (40 - 44) zum Entfernen des Referenzmusters aus dem Strahlengang, bzw. Mittel (4, 5, 11) zur zeitlichen Integration des aufgenommenen Bildes, vorgesehen sind, und dass das Speicher- und Rechnerwerk derart ausgebildet ist, dass die dreidimensionalen Daten zur Herstellung des Passkörpers jeweils dem zuletzt auf dem Monitor dargestellten referenzmusterfreien Videosuchbild entsprechen.

7. Vorrichtung nach Anspruch 6, dadurch gekennzeichnet, dass das Speicher- und Rechnerwerk eine Recheneinheit (52 - 55, 57, 58) aufweist, mittels welcher auf einen Auslösebefehl hin ein zum letzten Videosuchbild deckungsgleiches, um einen Falschlichtanteil korrigiertes Kontrastbild erzeugbar, speicherbar und auf dem Bildschirm (56) als Standbild darstellbar ist.

8. Vorrichtung nach Anspruch 7, dadurch gekennzeichnet, dass die Recheneinheit (52 - 55, 57, 58) derart ausgebildet ist, dass das Kontrastbild aus mehreren, zeitlich aufeinanderfolgenden Aufnahmen mit verschobenem Referenzmuster erzeugbar ist, um Streulicht und andere Störungen zu eliminieren, wobei das Kontrastbild deckungsgleich zu einem aus den Tiefenwerten gebildeten Relief ist.

9. Vorrichtung nach einem der Ansprüche 7 und 8, dadurch gekennzeichnet, dass die Recheneinheit auf zwei Speicherebenen (54, 55) zugreift, deren Speicherplätze je einem Bildpunkt entsprechen, wobei in der einen Speicherebene das Kontrastbild, in der anderen Speicherebene die zugehörigen Tiefenwerte speicherbar sind.

10. Vorrichtung nach Anspruch 9, dadurch gekennzeichnet, dass Mittel zur manuellen Eingabe und Speicherung von bezüglich dem Bildrahmen feststehenden Markierunen vorhanden sind, die dadurch den Bildebenen überlagert sind.

11. Vorrichtung nach Anspruch 9 oder 10, dadurch gekennzeichnet, dass eine in den Strahlengang der Kamera eingefügte optische Einrichtung (2 - 5, 40 - 44) zur Erzeugung des Licht- / Schatten-Referenzmusters auf der aufzunehmenden Präparation und eine optische Anordnung (7 - 9, 11) zur Abbildung derselben auf einen Bildsensor (11) vorgesehen sind, dadurch gekennzeichnet, dass mittels des Bildsensors (11) auf einen Auslösebefehl hin, jeweils bei räumlich verschiedener Lage des Musters, ein Helligkeitsbild detektierbar und jedes Helligkeitsbild in einer Speicherebene (54, 55) speicherbar ist, wobei aus den gespeicherten Helligkeitswerten mittels einer Schaltung (50 - 63) durch Transformation jeweils ein Wertepaar zu jedem Bildpunkt erzeugbar ist, dessen einer Wert den Abstand (z) des Bildpunktes zu einer Referenzebene und dessen anderer Wert die lokale Kontrastamplitude beinhaltet.

12. Vorrichtung nach einem der Ansprüche 6 bis 11, dadurch gekennzeichnet, dass das Licht- / Schatten-Referenmuster mittels einer im Strahlengang bewegbar angeordneten Maske (4) erzeugbar ist, dadurch gekennzeichnet, dass zur Erzeugung des Video-Suchbildes die Maske (4) synchron zur Bildaufnahmefrequenz bewegbar ist, so dass das Muster durch Integration verschwindet.

13. Vorrichtung nach einem der Ansprüche 6 bis 11, dadurch gekennzeichnet, dass das Licht- / Schatten-Referenmuster durch eine Flüssigkristallzelle (40 - 44) erzeugbar ist, die während der Erzeugung des Video-Suchbildes lichtdurchlässig geschaltet ist.

14. Vorrichtung nach Anspruch 11 oder 13, wobei das Referenzmuster durch eine Flüssigkristallzelle erzeugt wird, dadurch gekennzeichnet, dass die Flüssigkristallzelle (40 - 44) ein Elektrodenmuster aufweist, mittels welchem das Licht- / Schattenmuster um Bruchteile seiner Periode verschiebbar ist.

## Claims

1. Method for a three-dimensional imaging and representation of teeth, respectively of worked surfaces of the same and of their immediate neighbourhood, with the aid of a camera, for producing three-dimensional data for the manufacture of an insert body, where a reference pattern is projected in order to obtain depth information, characterized in that in order to determine an adequate viewing angle of the camera a moving video monitoring image, in which the superimposed projected pattern is not shown, is first generated and displayed on a monitor, and that upon receipt of a triggering signal a sequence of images is taken for generating and storing a set of data which on the one hand contains image information that corresponds to the last search image and can be represented as a contrast image on the monitor in order to be used as a base for drawing additional details, and which on the other hand contains depth values which are localized in correspondence with the image, so that an additional drawing made on the contrasted image defines the three-dimensional insert when taken in conjunction with the depth values.

2. Method according to claim 1, characterized in that for the obtention of a still image the contrast values, ie the light values, corrected for an undesirable secondary illumination, are imaged for each pixel, in order to generate a pseudosolid three-dimensional representation of the worked surface.

3. Method according to one of the preceding claims, characterized by the insertion of markers, which are fixed with respect to the image frame and which correspond to the stored image information, so that the video search image, respectively the data to be registered, can be adjusted with respect to the inserted markers.

4. Method according to one of the preceding claims, characterized in that one uses the still image as the image plane for the computer-aided addition of contour lines for the insert, where the contours of the insert body which are entered are complemented by pairing image points of the still image with corresponding depth values, in order to obtain a three-dimensional contour design.

5. Method according to any of claims 2 to 4, characterized in that for a further processing the depth values are being weighted with the help of the corresponding contrast values, so that the statistical value of each depth value depends on the strength of the corresponding contrast.

6. Device for implementing the method of any of the preceding claims, with a memory and a calculating unit (51 - 55, 57 - 63) , which determines the three-dimensional information for manufacturing the insert body from the registered image signals, by using the movable reference pattern projected in superimpression, characterized in that in order to suppress the reference pattern for a pattern-free representation of the monitoring video image on the monitor (56) means (40 - 44) for eliminating the reference pattern from the ray path, respectively means (4, 5, 11) for a time-integration of the registered image, are provided, and in that the memory and the calculation unit are so constructed that the three-dimensional data for manufacturing the insert body always correspond to the last pattern-free monitoring video image which was represented last on the monitor.

7. Device according to claim 6, characterized in that the memory and calculating unit comprises a calculating element (52 - 55, 57, 58) which, when activated by a trigger signal, is able to generate, store and represent in form of a still image on the monitor screen (56) a contrasted image which is in one-to-one correspondence with the last video image and which is corrected for unwanted secondary illumination.

8. Device according to claim 7, characterized in that the calculating element (52 - 55, 57, 58) is such that the contrasted image can be generated from several pictures taken in succession and with a shifted reference pattern, in order to eliminate reflected light and other artefacts, the contrasted image being in one-to-one correspondence with a relief generated from the depth values.

9. Device according to one of claims 7 and 8, characterized in that the calculating unit has access to two memory planes (54, 55), where any memory location of each corresponds to one image pixel, the contrasted image being stored in one memory plane, and the corresponding depth values in the other.

10. Device according to claim 9, characterized in that it comprises means to manually input and to store markers which are fixed relative to the image frame, and thereby superimposed on the image planes.

11. Device according to claim 9 or 10, characterized in that it comprises optical means (2 - 5, 40 - 44) introduced in the ray path of the camera in order to generate the reference pattern of light and shadows on the worked surface, and an optical device ( 7 - 9, 11) for imaging the same on an image sensor (11), characterized in that an optical image can be detected through the image sensor (11), upon a triggering signal occurring for different positions of the pattern, that each optical image can be stored in a storage plane (54, 55), whereby a circuit (50 - 63) generates, from the stored light values and by way of a transformation, a pair of values for each image point, one value of which represents the distance (z) of the image point to a reference plane, and the other value of which represents the local amplitude of the contrast.

12. Device according to any of claims 6 to 11, characterized in that the reference pattern of light and shadows can be generated through a mask (4) movably mounted in the ray path, characterized in that in order to generate the monitoring video image the mask (4) is movable synchronously with the image-taking frequency, such that the pattern is suppressed by integration.

13. Device according to any of claims 6 to 11, characterized in that the reference pattern of light and shadows can be generated by a liquid crystal cell (40 - 44) which is commanded so that it lets light pass when the video image is being generated.

14. Device according to claim 11 or 13, where the reference pattern is generated by a liquid crystal cell, characterized in that the liquid crystal cell (40 - 44) comprises an electrode pattern able to shift the pattern of light and shadows by fractions of its period.

## Revendications

1. Procédé pour la prise de vue tridimensionnelle et la représentation de dents ou respectivement de leurs préparations et de leur voisinage immédiat au moyen d'une caméra pour la production de données tridimensionnelles pour la fabrication d'une pièce d'adaptation, un modèle de référence étant projeté pour la production des données de profondeur, caractérisé en ce que, pour déterminer la position de prise de vue appropriée de la caméra, on forme tout d'abord une image de localisation vidéo déplacée et on la visualise sur un moniteur sur lequel n'apparaît pas le modèle de référence projeté et que, sur un ordre de déclenchement en position de prise de vue, une séquence de prise de vue d'images se déroule pour la formation et la mémorisation d'un article qui contient d'une part une information d'image qui correspond à la dernière image de localisation et qui peut être visualisée sur le moniteur sous forme d'une image de contraste en tant que modèle de dessin et qui contient d'autre part des valeurs de profondeur coincidentes pour l'information d'image, de sorte qu'une construction réalisée sur le modèle de dessin définit avec les valeurs de profondeur la pièce d'adaptation en trois dimensions.

2. Procédé selon la revendication 1, caractérisé en ce qu'on représente en tant qu'image fixe les valeurs de contraste, c'est-à-dire les luminosités corrigées d'une fraction de lumière parasite, en chaque point d'image, pour former une représentation pseudo-plastique de la préparation.

3. Procédé selon l'une des revendications précédentes, caractérisé en ce qu'on mélange dans l'image de localisation vidéo des repères fixes par rapport au cadre d'image, qui correspondent à des données d'image mémorisées, de sorte que l'image de localisation vidéo ou respectivement l'article à prendre en vue peut être aligné par rapport aux repères mélangés.

4. Procédé selon l'une des revendications précédentes, caractérisé en ce qu'on utilise l'image fixe comme plan de figure pour la construction de contours de pièce d'adaptation à l'aide de moyens de dessin électroniques, les contours de pièce d'adaptation introduits étant étendus à des données de contour tridimensionnelles sur la base de l'adjonction des points d'image de l'image fixe aux valeurs de profondeur correspondantes.

5. Procédé selon l'une des revendications 2 à 4 précédentes, caractérisé en ce qu'on pondère les valeurs de profondeur à l'aide des valeurs de contraste associées pour le traitement ultérieur, de manière que le poids statistique de chaque valeur de profondeur est dépendant de la grandeur de la valeur de contraste correspondante.

6. Dispositif pour la mise en oeuvre du procédé selon l'une des revendications précédentes, un compteur-calculateur (51-55, 57-63) étant prévu qui détermine, des signaux d'image pris en vue à partir du modèle de référence translatable projeté, les données tridimensionnelles pour la fabrication de la pièce d'adaptation, caractérisé en ce que, pour l'élimination du modèle pour la représentation sans modèle de référence de l'image de localisation vidéo sur le moniteur (56), des moyens (40-44) pour éliminer le modèle de référence de la marche des rayons, ou respectivement des moyens (4, 5, 11) pour l'intégration temporaire de l'image prise en vue sont prévus, et que le compteur-calculateur est réalisé de telle manière que les données tridimensionnelles pour la fabrication de la pièce d'adaptation correspondent respectivement à l'image de localisation vidéo sans modèle de référence représentée en dernier lieu sur le moniteur.

7. Dispositif selon la revendication 6, caractérisé en ce que le compteur-calculateur comprend une unité de calcul (52-55, 57, 58) au moyen de laquelle, sur un ordre de déclenchement, une image de contraste corrigée d'une fraction de lumière parasite, coïncidente à la dernière image de localisation vidéo, peut être formée et mémorisée et peut être représentée sur l'écran (56) en tant qu'image fixe.

8. Dispositif selon la revendication 7, caractérisé en ce que l'unité de calcul (52-55, 57, 58) est réalisée de telle manière que l'image de contraste peut être formée à partir de plusieurs prises de vue se suivant les unes des autres dans le temps avec modèle de référence décalé, afin d'éliminer la lumière diffusée et d'autres parasites, l'image de contraste étant coïncidente à un relief formé à partir des valeurs de profondeur.

9. Dispositif selon l'une des revendications 7 et 8, caractérisé en ce que l'unité de calcul accède à deux étages de mémoire (54, 55) dont les emplacements de mémoire correspondent chacun à un point d'image, l'image de contraste pouvant être mémorisée dans l'un des étages de mémoire et les valeurs de profondeur associées dans l'autre étage de mémoire.

10. Dispositif selon la revendication 9, caractérisé en ce que des moyens pour l'introduction et la mémorisation manuelles de repères fixes par rapport au cadre d'image sont disponibles, qui sont superposés par eux aux plans d'image.

11. Dispositif selon la revendication 9 ou 10, caractérisé en ce qu'un dispositif optique (2-5, 40-44) inséré dans la marche des rayons pour former le modèle de référence en lumière/ombre sur la préparation à prendre en vue et une structure optique (7-9, 11) pour la représentation de celui-ci sur un capteur d'image (11) sont prévus, caractérisé en ce que, au moyen du capteur d'image (11), sur un ordre de déclenchement, respectivement pour une position différente dans l'espace du modèle, une image de luminosité peut être détectée et chaque image de luminosité peut être mémorisée dans un étage de mémoire (54, 55), une paire de valeurs pour chaque point d'image pouvant être formées respectivement à partir des valeurs de luminosité mémorisées, au moyen d'un couplage (50-63) par transformation, dont une des valeurs contient la distance (z) du point d'image à un plan de référence et l'autre valeur l'amplitude de contraste locale.

12. Dispositif selon l'une des revendications 6 à 11, caractérisé en ce que le modèle de référence en lumière/ombre peut être formé au moyen d'un masque (4) disposé mobile dans la marche des rayons, caractérisé en ce que, pour la formation de l'image de localisation vidéo, le masque (4) est mobile en synchronisme avec la fréquence de prise de vue, de sorte que le modèle disparaît par intégration.

13. Dispositif selon l'une des revendications 6 à 11, caractérisé en ce que le modèle de référence en lumière/ombre peut être formé par une cellule à cristaux liquides (40-44) qui est commutée en transparence à la lumière pendant la formation de l'image de localisation vidéo.

14. Dispositif selon la revendication 11 ou 13, le modèle de référence étant formé par une cellule à cristaux liquides, caractérisé en ce que la cellule à cristaux liquides (40-44) présente un modèle d'électrode par lequel le modèle en lumière/ombre peut être décalé de fractions de sa période.

**SUCHPHASE**

KAMERA WIRD EXAKT AUSGERICHTET

VIDEO SUCHBILD

**AUFNAHME & TRANSFORMATION**

KAMERA BLEIBT IN POSITION

**KONTROLLE**

AUFNAHME GUT ?

NEIN — JA

KONTRASTBILD DARGESTELLT

**KONSTRUKTION DES PASSKÖRPERS**

AUFNAHME WIRD AM KONTRASTBILD ERGÄNZT. ZUGEORDNETE TIEFEN DEM RELIEF ENTNOMMEN

ÜBERLAGERTES RELIEF IM SPEICHER

*Fig 1.*

(HAUPTDARSTELLUNG)

**Fig. 2**

ANSTEUERUNG A,C,D

ANSTEUERUNG B

**Fig 4.**

3a

3b

3c

3d

*Fig. 5*